# EUROPEAN PATENT APPLICATION

(11) **EP 4 686 476 A1**
(43) Date of publication of application: **04.02.2026**
(21) Application number: 24192168.3
(22) Date of filing: 31.07.2024
(51) Int. Cl.: A61K 31/553, A61P 25/00, A61P 25/16, A61P 25/28

(54) **PHOSPHOINOSITIDE 3 KINASE (PI3K) INHIBITOR FOR USE IN THE TREATMENT OF NEUROLOGICAL DISEASES**

(71) Applicant: Klostermeier, Stefanie, 81541 München (DE)
(72) Inventor: Klostermeier, Stefanie, 81541 München (DE)
(74) Representative: Fleuchaus & Gallo Partnerschaft mbB

(57) **Abstract**

The invention refers to the field of neurodegenerative diseases including Alzheimer's Disease and a treatment thereof with a Benzoxazepine compound. Benzoxazepine compounds are e.g. known in the treatment of breast cancer and according to the use of the invention are a first therapeutic and prophylactic treatment for neurodegenerative diseases, including Alzheimer's Disease.

The invention further relates to test systems for identifying, mapping, elaborating and evaluating said and further therapeutic and prophylactic uses of compounds of interest and/or other pharmaceutical compositions for the treatment of neurodegenerative diseases, including Alzheimer's Disease. (AD)

## Description

The invention refers to the field of Alzheimer's Disease (AD) and further neurological disorders and provides a new treatment approach for such neurological disorders and AD. For the treatment of neurological disorders is thus provided PI3K inhibitors and particularly provided are P13K-inhibitors identifiable as Benzoxazepine or compositions thereof. Benzoxazepine compounds are known and approved for use in the treatment of certain types of breast cancer.

Additionally, the invention is based on a brain-specific test systems capable of simulating physiological and pathological conditions of the human brain. This system has been used for testing, identifying, mapping, elaborating and evaluating substances for their effects on the brain.

As one result, identified with the test system, PI3K inhibitors were identified for their therapeutic and/or prophylactic use in the treatment of neurological disorders, beside others for dementia, Alzheimer's Disease (AD), vascular Dementia, Down syndrome (DS) or diabetic Polyneuropathy.

Alzheimer's disease will be one of the major challenges of the century. Presently every third person above the age of 90 is affected, every seventh person above the age of 80 is affected. The global situation shows an incidence, that has increased from 1990 to 2019 by around 145%, resulting in more than 7 million cases recorded worldwide in 2019. Considering the predictable demographic development this sums up to more than 150 million Alzheimer patients in 2050. As there is no treatment, not only the families that have the challenge of caretaking but also society will dramatically be affected. So far, medicine and research are hampered as only unsuitable test systems or animal models seems available or feasible for scientific analysis of early stages of Alzheimer.

Presently, there is not yet a reliable test system that allows the testing of drugs or medicaments suitable for the treatment of neurological disorders and in particular of Alzheimer, neither is there any transferability of results from any such systems to humans and the pathology of Alzheimer's disease, which can be extracted from the high failure rate of preclinical promising results of AD drugs in clinical trials in humans.

Additionally, it is morally unacceptable and scientifically questionable to test potential drug for such diseases and Alzheimer on healthy humans. Consequentially, as an initial testing of newly developed drugs in humans is not acceptable, but at the same time typically all clinical phase 1 studies are trials where new drugs are tested in humans, after that they are extensively tested in two different animal species, this lack of test systems or suitable animal models systematically blocks the development of new drugs for neurological disorders or particularly Alzheimer's disease.

Another pressing challenge in Alzheimer's disease research is that the typical time progression to develop the disease takes several decades. A lack of suitable test systems, e.g., biomarkers, to identify the Alzheimer's disease patient cohort at least 5 to 10 years prior to the initial Alzheimer's disease state, which is described as "mild cognitive impairment", makes it even more challenging to identify or test high potential drug candidates that can treat the disease early on.

Even if one would want to test potential drugs for the treatment on Alzheimer, clinical trials sound impossible, as presently it is impossible to collect data in AD typical timeframes. Even if, patients - known to be at risk to develop Alzheimer - are treated, any result whether they actually develop Alzheimer or whether an AD development can be stopped and whether an actual Alzheimer disease did only develop into a less severe status, would need to wait at least for 5 to 10 years to decribe the effects of such treatment. Worse, certainly we can hardly make any correlation between a treatment and a result that is analyzed in the brain tissue years later or only in a deceased patient years later.

It is to the credits of the inventor, that there is now a research tool suitable to address this need and to simulate the physiological and pathological situations for human brain cells. With the new test system, it is now possible to analyze, identify, classify, quantify, observe and evaluate the effect of pharmaceutical drugs or other blood-brain-barrier transmittable components on neurons, glia cells, e.g., microglia and astrocytes and all the other cellular, extracellular or intracellular functions in the brain, which are involved in the development and/or progression of neurological diseases such as Alzheimer's disease.

Accordingly, the invention refers to the treatment of neurodegenerative diseases including Alzheimer's Disease and a treatment thereof with a Benzoxazepine compound. Benzoxazepine compounds are with under known to be used for the treatment of breast cancer and according to the use of the invention are a first therapeutic and prophylactic treatment approach for neurological and neurodegenerative diseases, such as Alzheimer's disease.

The invention further illustrates how the new test systems will be used to analyze, identify, map, elaborate and evaluate further therapeutic and prophylactic uses of compounds of interest and/or other pharmaceutical compositions for and in the treatment of neurological and neurodegenerative diseases, including Alzheimer's Disease (AD).

Though, in the following sections the treatment of AD is used as an example, however other neurological or neurodegenerative diseases and disorders as well as other suitable drugs to treat them can be identified with this invention.

Neurodegenerative diseases and disorders suitable for such treatment approaches include e.g. Frontotemporal dementia, Dementia with Lewy bodies, Parkinson's disease, Huntington's disease, Progressive supranuclear palsy, Corticobasal degeneration, Multisystem atrophy and Progressive myoclonic epilepsy's and many more as later explicitly mentioned.

Alzheimer's disease (AD) is a major cause of suffering and death globally, and with 60-70% of all 9.9 million new estimated Dementia cases each year the largest part among the humans directly suffering from Dementia and accompanying complications. AD is a slowly progressive neurodegenerative disease, which goes along with dementia, loss of memory, loss of decision making, loss of orientation, significant morbidity, multiple comorbidities and consequentially leading to a major loss of quality of life for the patient but also her/his family or responsible care takers (Barker *et al*., (2020)).

In the next decade AD will rapidly become an increasingly severe problem for societies all over the world. A prediction states, that in 2030, the number of Americans diagnosed with AD will have experienced an increase of 35%. The global situation shows an incidence, that has increased from 1990 to 2019 by 147,95%, resulting in 7,24 million cases recorded in 2019. In 2050 the incidence of AD is projected to reach 152,8 million cases worldwide.

Scientific reviews such as Barker *et al*., (2020) summarize the clinical picture including the symptoms and progression of AD development, which typically starts with a loss of short-term memory, later follows cognitive failure and confusion, and finally an inability to carry out tasks required for successful daily living.

Despite immense efforts for better understanding AD, it must be accepted that the cause of AD onset is still unclear. There are several hypothesizes proposed to explain the pathophysiology of AD. One established hypothesis considers the development of plaques due to the accumulation of A-beta-42-amyloid-peptides (Aβ) within the extracellular matrix of brain- and CNS-tissue as the root cause of AD. Another hypothesis speculates about the hyperphosphorylation of Tau protein (pTau), being intracellular neurofibrillary tangles, as possibly driving pathomechanism underlying AD.

It is further believed that these two driving forces are interconnected and thatAβ-accumulation increases intracellular hyperphosphorylation of pTau within neurons and/or within the brain- and CNS-tissue. According to Barkers *et al.,* the accumulation and hyperphosphorylation are causally correlated with the death of neurons and consequentially the AD symptoms.

Additionally, and confirming the existence of a genetic predisposition for AD, which triggers an even earlier onset of AD, comparative studies between healthy patients and AD patients have helped to identify AD risk genes, of which it is assumed that their expression is correlated with or maybe even causes the onset of AD. Presently known examples thereof are e.g.: BLNK, ADAM10, ADAM17, TREM2, RhoH, and SIGLEC11.

This list is however not to be understood as a complete list, and more recent studies also identify the PI3K cascade and genes participating in this cascade as possibly being involved in the pathology of AD. Barker *et al*. (2020) proposed the PI3K pathway as one explanation for the pathophysiology of AD.

While Barker *et al*. focusses on a potential role of the PI3K-pathway in AD, the paper also mentions the participation PI3K pathway in cancer, particularly breast cancer. Disturbingly, it seems that PI3K involvement in AD and cancer is quite contradictory and seems to trigger very distinct and unequal effects of dysregulation in breast cancer or potentially in AD. Barker *et al*. summarizes that most cancers, including breast cancer, involve or are caused by unrestrained cell proliferation. For breast cancer, it was found that such cell proliferation often coexists with a PI3K mutation. In those so-called PI3Kinase-dependent or-associated forms of breast cancer there is found a hyperactivity of the PI3K/Akt signaling pathway, which then leads to the cancerogenic dysregulation of cell proliferation.

The situation within the brain or brain cells, such as neurons and glia, e.g., microglia or astrocytes, the PI3K activity and/or regulation seems to be different. For example, for neurons it is believed that they remain in a metabolic stable status, meaning neither do they proliferate anymore, nor do they grow any further. Thus, it can be said under healthy conditions, neurons remain in stable state, and even though they may express receptors, which in many other cells would lead to cell growth, they will not actively proliferate. Neurons in the brain, are terminally differentiated post-mitotic cells, which if forced into a renewed cycle usually die.

It is exactly such a hypothesis, which nicely illustrates the actual problem of AD research starts. As there is no suitable animal model for studying AD and as it is unacceptable to randomly treat patients of which one only assumes that they might - one day - develop AD, any such speculation regarding a potential involvement of e.g. the PI3K pathway in AD cannot properly be tested.

It is due to the efforts and development of the inventor that a novel method and system for testing the effect of high potential drug candidates and potentially interesting substances or drugs for treatment of neurons, microglia and astrocytes were established. With this method and system, the inventor was capable to newly identify suitable drugs that lead to a significant reduction of protein concentrations of e.g., Aβ and pTau by interfering with and/or inhibiting the PI3K pathway.

For the first time it is possible on the basis of this system to not only observe the development of the pathophysiology of AD (described in Klostermeier *et al*., *- in press)* but - even more interesting - to identify substances and/or compositions, which interfere, also reduce and delay or even may revert the pathophysiology of AD.

The present invention thus solves two major problems that have for decades restrained the science and research of neurogenerative diseases and the development of suitable treatments for neurogenerative diseases. On the one hand the inventor identified a new and previously unknown hypothesis for the development and progression of AD, and on the other hand - overcoming the restrictions of the past - the inventor here provides the groundbreaking idea of how a substance or composition must act to effectively reduce the risk of AD or even cure AD.

Accordingly, and thus solving the above-mentioned problems, the inventor provides a system that allows to simulate and to observe the interaction between different brain cells not only in physiological, but also in pathophysiological conditions. In this system it is now possible to test and thus better understand previously suggested hypothesis regarding the onset or development of neurogenerative diseases and/or AD.

The discovery of the inventor that an essential involvement in the development of AD or more general in the development of neurological and neurodegenerative diseases is caused by changes of tissue's mechanobiology in the brain ((*in press* - *Klostermeier et. al.,*) is used in the herein provided system to firstly show the pathological development and to thereafter the system is used to identify substances which can stop and/or reduce such pathological development.

The herein presented teaching of the inventor for the treatment of AD in the future allows a treatment regime, which is capable to effectively reduce the protein concentrations of Aβ and pTau firstly in the system but correspondingly also in the human brain. The inventor shows that by inhibiting the PI3K pathway the expression of the Aβ and pTau gene expression or more generally spoken the Aβ and pTau protein accumulation an effective reduction of these toxic proteins and, thus, a reduction of the accumulation and the formation of Aβ plaques and hyperphosphorylated pTau in the brain is obtained.

Accordingly, the inventive treatment approach focuses in its most general embodiment on the functional inhibition of the PI3K pathway. Additionally, as one result of employing the new test system the inventor could identify a PI3K inhibitor as a drug suitable to be used in the treatment of Alzheimer's disease. Thus, as a result of employing the new system and method the inventor could identify a drug suitable to be used in the treatment of neurological disorders, particularly AD.

The invention provides the class of PI3K-inhibitors for use in the treatment of neurological disorders. In the context of the invention the term **"neurological disorder"** refers to or identifies pathophysiological condition concerning the brain and primarily cells, such as neurons and glia cells in the brain, but also pathophysiological condition of e.g. neurons in the periphery. Furthermore, the term neurological disorder includes neurodegenerative diseases, neuropathological disorders and polyneuropathies.

Examples of **"neurodegenerative diseases"** include, but are not limited to, Alzheimer's disease (AD), Amyotrophic Lateral Sclerosis, Dementia, Vascular dementia, Frontotemporal dementia, Dementia with Lewy bodies, Parkinson's disease, Huntington's disease, Progressive supranuclear palsy, Corticobasal degeneration and Multisystem atrophy.

The term **"neuropathological disorder"** refers to or describes diseases and/or disorders of the central nervous system and/or the peripheral nervous system, optionally, but not necessarily, including the muscles. Examples of neuropathological disorders include, but are not limited to genetic diseases, amongst others Down Syndrome, migraine, inflammatory diseases, inflammatory bowel diseases, Crohn's disease, ulcerative colitis, meningitis, encephalitis, Creutzfeldt-Jakob disease, neuroborreliosis, multiple sclerosis, neuromyelitis optica, Guillain-Barré syndrome, rheumatoid arthritis, systemic lupus erythematosus, epilepsies and seizure disorders, progressive myoclonic epilepsy, traumatic brain injuries, spinal cord injuries, nervous system tumors, brain tumors, spinal cord tumors, peripheral nerve tumors, glioma, glioblastoma, neuromuscular disorders such as myasthenia gravis, muscular dystrophies and spinal muscular atrophy.

The term **"polyneuropathy"** refers to or describes an impairment of all parts of the nervous system. An example of polyneuropathies includes, but is not limited to, diabetic polyneuropathy.

In the context of the invention the terms **"treatment"** and **"treat"** refer to or describe both therapeutic treatment and prophylactic or preventative measures, wherein the object is to prevent or slow down (lessen) an undesired neurological disorder, such as neurodegeneration. For purposes of this invention, beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Those in need of treatment include those already with the condition or disorder as well as those prone to have the condition or disorder or those in which the condition or disorder is to be prevented.

In the context of the invention the term **"PI3K"** refers to or describes a group of enzymes known as **"P13-Kinases",** also referred to as Phosphatidylinositol3-kinases. Phosphatidylinositol3-kinases (PI3Ks) are **lipid kinases** capable of phosphorylating the 3'-hydroxyl group of the inositol ring of phosphatidylinositol. PI3Ks integrate signals from growth factors, cytokines and other extracellular stimuli into intracellular signals to regulate cell growth, proliferation, survival, motility and metabolism.

Furthermore, the PI3K pathway is an extracellularly started intracellularly proceeding signaling cascade. Under physiological conditions it starts with the binding of an activating ligand on the transmembrane PI3K-Receptor (PI3K), leading also to the change of the intracellular part of PI3K pathway and - it is believed - eventually to the accumulation of hyperphosphorylated pTau in neurons.

In the context of the invention the term **"PI3K-inhibitor"** refers to or describes substances, compounds or compositions, which themselves and/or in their activated form inhibit PI3K, i.e. slow down, prevent or stop in a concentration-dependent manner the enzymatic activity of PI3K.

By providing therapeutically active concentrations of the PI3K inhibitor to a patient the PI3K-cascade can be slowed down, interrupted or even stopped. Thus, a treatment with a P13K-inhibitor advantageously reduces the negative effects transmitted by an activated PI3K-cascade on brain cells or other neuronal cells and supports a physiological neuroplastic restructuring.

Therefore, this invention provides **a substance suitable as first in class** for use in the treatment of neurological disorders.

According to the invention herewith provided is a substance or composition of Formula (I) for the use in the treatment of a neurological disorder, neurogenerative disease or disorder, and among others Alzheimer's Disease. 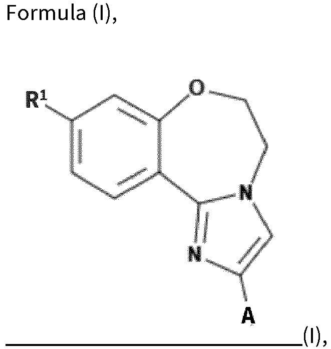 which also can be identified as Benzoxazepine.

Such Benzoxazepine are known in the art and particularly from WO2011036280 or WO2013182668 where they are used in the treatment of cancer, in particularly in a specific breast cancer having mutations in the PI3K pathway, which cause tumor growth and metastasis.

According to one embodiment Formula (I) is characterized by a first residue R¹ and the second residue A, the first residue R¹ and the second residue A selected from a group of substituents as identified below and thus, leading to the known compounds GDC-0032 and GDC-0077.

In one preferred embodiment of the substance or composition of Formula (I) for the use according to the invention is defined by the residue R¹ being a 1H-pyrazol-1-yl-2-methylpropanamide, as shown 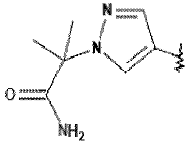 and the second residue A being a 1-isopropyl-3-methyl-1H-1,2,4-triazol-5-yl, as shown 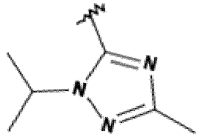 leading to an Benzoxazepine compound of Formula (II), namely

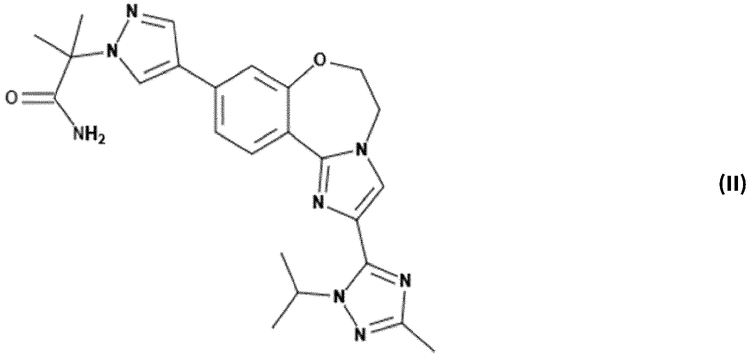

The Benzoxazepine compound of Formula (II), and it's usability for the treatment of cancer in humans is known from EP2858666B1. The Benzoxazepine compound of Formula II is also known as **GDC-0032,** 2-(4-(2-(1-isopropyl-3-methyl-1H-1,2,4-triazol-5-yl)-5,6-dihydrobenzo[f]imidazo[1,2-d][1,4]oxazepin-9-yl)-1H-pyrazol-1-yl)-2-methylpropanamide **or Taselisib** (INN).

The Benzoxazepine of Formula (II), is believed to be an unspecific PI3K-inhibitor, i.e. binding the various PI3Ks. The provision of an unspecific PI3K-inhibitor, namely the Benzoxazepine compound of Formula (II), is particularly advantageous as a first in class substance for the treatment in neurological disorders and thus, is a good starting point for further comparison with other compounds regarding their effect, efficiency in reducing the expression of target genes or the reestablishing of physiological conditions in brain cells.

In a second preferred embodiment of the substance or composition of Formula (I) for the use according to the invention is defined by the residue R¹ being a (S)-2-(2-amino)propenamide, as shown 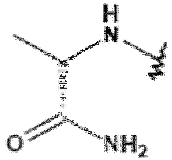 and the second residue A being a (S)-4-(difluoromethyl)-2-oxooxazolidin as shown 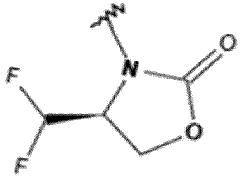 leading to an Benzoxazepine compound of Formula (III)

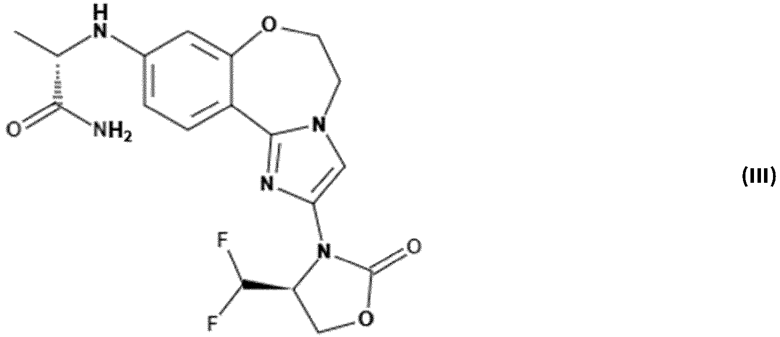

The Benzoxazepine compound of Formula (III), and it's usability for the treatment of cancer in humans is known from WO 2020023297A1. The Benzoxazepine compound of Formula (III) is also known as **GDC-0077,**

(S)-2-((2-((S)-4-(difluoromethyl)-2-oxooxazolidin-3-yl)-5,6-dihydrobenzo[f]imidazo[1,2-d][1,4]oxazep in-9-yl)amino)propenamide or as **Inavolisib** (=INN).

What is remarkable about this specific Benzoxazepine compound of Formula (III) is, it's selectivity in inhibiting the PI3Kα isoform with higher affinity as the PI3Kβ, PI3Kδ, and Pi3Kγ isoforms.

Furthermore, according to the invention also stereoisomer, constitutional isomer, geometric isomer, tautomer or pharmaceutically acceptable salt of the Formula (I), (II) or (III) are provided for the treatment of neurological disorder, neurogenerative disease or disorder and Alzheimer's Disease.

In the context of the invention the term **"pharmaceutically acceptable salt",** refers to or describes pharmaceutically acceptable organic or inorganic salts of a compound of the invention. They must be compatible chemically and/or toxicologically, with the other ingredients comprising a substance or composition. Exemplary pharmaceutically acceptable salt include, but are not limited to sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucuronate, saccharate, formate, benzoate, glutamate, methanesulfonate "mesylate", ethanesulfonate, benzenesulfonate, p-toluenesulfonate, and pamoate (i.e., 1,1'-methylene-bis-(2-hydroxy-3-naphthoate)) salts. A pharmaceutically acceptable salt may involve the inclusion of another molecule such as an acetate ion, a succinate ion or other counter ions. The counter ion may be any organic or inorganic moiety that stabilizes the charge on the parent compound. Furthermore, a pharmaceutically acceptable salt may have more than one charged atom in its structure. Instances where multiple charged atoms are part of the pharmaceutically acceptable salt can have multiple counter ions. Hence, a pharmaceutically acceptable salt can have one or more charged atoms and/or one or more counter ions.

According to the invention the substance or composition is used in the treatment of the neurological disorder selected from the group of neurological disorders comprising neurodegenerative diseases and neuropathological disorder, including but not limited to Alzheimer's disease, Amyotrophic Lateral Sclerosis (ALS), Dementia, Vascular dementia, Frontotemporal dementia, Dementia with Lewy bodies, Parkinson's disease, Huntington's disease, progressive supranuclear palsy, corticobasal degeneration, migraine, multisystem atrophy, Down syndrome (DS), inflammatory bowel diseases, Crohn's disease, ulcerative colitis, meningitis and encephalitis, migraine, Creutzfeldt-Jakob disease and neuroborreliosis (Lyme disease), multiple sclerosis, neuromyelitis optica, Guillain-Barré syndrome, rheumatoid arthritis, systemic lupus erythematosus, epilepsies or seizure disorders, progressive myoclonic epilepsy, traumatic brain injuries, spinal cord injuries, neuromuscular disorders, myasthenia gravis, muscular dystrophies, spinal muscular atrophy, polyneuropathies and diabetic polyneuropathy.

The substance or composition according to the invention is particularly advantageous for the use in those diseases, caused by, related to, accompanied with and/or following a neuroplasticity rearrangement, disorders or trauma. Genetic predisposition can trigger or increase the risk for a neurological or neuropathological disorder.

In another certain embodiment the substance or composition according to the invention is administered oral, intravenous, subcutaneous, intrathecal, sublingual, transbuccal, transdermal, nasal, pulmonal, rectal, as Antibody-Drug-complex and/or as liposomal nanoparticles.

According to this further embodiment the galenic formulations of the substance or composition of the invention is relevant, particularly when treating disorders of the brain and in the brain.

The brain, as separated from the peripheral immune system by the blood-brain-barrier (BBB) relies also on the innate immune system for defense. It is believed that part of this innate immune response includes production of Aβ-peptide and activation of the resident macrophages, glia, resulting in neuroinflammation, neuronal loss and ultimately death. Unless constantly cleared from the extracellular brain space such Aβ formed plaques are toxic, insoluble oligomeric forms, which may cause neuroinflammation, trigger phagocytic activity of microglia cells and contribute to neuronal death.

For a transfer through the BBB the skilled practitioner may provide suitable pharmaceutical formulations. Alternatively, the skilled practitioner may use complexes of the substance of the invention with BBB crossing antibodies, likely single chain antibodies, forming so-called Antibody-Drug-Complexes (ADC), which are known to transport cargo molecules into the brain.

In another embodiment the substance or composition according to the invention, is administrable as a retard composition. The term "retard composition" refers to or describes a formulation of the compound or composition designed for a slow or delayed release.

Interestingly, it is shown in the examples, that not only the dose, which is used in the treatment of breast cancer but also a substantially lower dose is significant and effectively modifying the metabolism of and gene expression in brain cells. Experimental data show, that already a dose of 10 nM (in the Figure the abbreviation nm was used for nanomolar) is sufficient for a drastic reduction of the protein concentration and thus a potentially curing effect.

The term "therapeutically effective amount" refers to or describes a selectable concentration of a pharmacologically active substance of Formula (I), (II), (III) and/or a pharmacologically active metabolite of said substances respective to time and body weight.

In another embodiment the substance or composition according to the invention is used as a prophylactic or post-expositional treatment. In the context if the invention the term "prophylactic" describes or relates to an administration of the substance of the invention after a first notice of a risk factor for developing e.g. neurological disorder. Alternatively, a prophylactic use also is indicated where there is a genetic predisposition for the development of a neurological disorder or any of the above-mentioned specific diseases. The term "post-expositional" describes or relates to an immediate administration after exposure to an acute risk-factor for developing the neurological disorder, which can be a trauma e.g. induce by an internal bleeding, concussion, injury, the application of brute force on the head or similar incidences.

Such prophylactic or post-expositional treatment with the substance or composition of the invention, which stabilized the metabolism of the affected brain cells, is expected to be particularly useful for avoiding long-term brain damage.

The substance of the present invention has been identified in **a 3D cell culture system** for identifying and testing substances and/or compositions of interest for their effect on brain cells, wherein the 3D cell culture system is suitable for the cultivation of one or more species of neuronal cells or brain cells, wherein the 3D cell culture system comprises at least two different cell cultivation hydrogels made from Alginate-dialdehyde (ADA) and collagens.

The at least two hydrogels, i.e. the at least first and at least second hydrogel are made according to the Examples of ADA and collagen, for the present testing ADA PH124 and PH176 (VIVAPHARM JRS Pharma, Ulm, Germany) were used for the Sysnthesis as described in Example 2. These ADAs, namely PH124 und PH176 lead to the differences in mechano stability and viscoelasticity of the resulting hydrogels that resembles the human healthy and pathological brain.

The 3D cell culture system and test system according to the invention is characterized by the use of hydrogels with such individually distinct biomechanics, which are adjustable while preparing the hydrogels.

Explicitly, and for testing brain specific drug candidates the at least first and at least second hydrogel have defined and distinct stiffness and elasticity. Together the stiffness and elasticity of the individual hydrogel can be described and is defined by its viscoelasticity.

In the context of the present invention the term "viscoelasticity" describes the property of materials that exhibit both viscous and elastic characteristics when undergoing deformation. Viscoelastic materials have time-dependent strain, meaning they exhibit both immediate elastic response and delayed viscous response under stress.

Further in this context the term "viscous" or "viscosity" refers to a measure of a fluid's resistance to flow. It describes the internal friction within the fluid that arises when adjacent layers move at different speeds. For biomaterials, viscosity can describe how the material resists deformation under shear stress.

Still further in this context the term "elastic" or "elasticity" refers to the property of a material to return to its original shape after the removal of a force that caused deformation. In biomaterials, elasticity is critical in understanding how tissues and other biological materials respond to mechanical forces.

This viscoelasticity of the individual hydrogels is individually designed depending on the test situation and purpose of the 3D cell culture system.

One way to design the individual hydrogels of the 3D cell culture system is to mimic with the specific viscoelasticity in the at least first hydrogel the physiological conditions of a certain organ, e.g. of healthy brain tissue. In the corresponding at least second hydrogel would then be mimicked a viscoelasticity of amended conditions for said organ, and in the example with the brain such at least second hydrogel has then the viscoelasticity of a pathological brain condition.

Such corresponding hydrogels with distinct and different viscoelasticity are then populated with the test cells, which subsequently can be exposed to a substance of interest for testing the effects on these cells under distinct, namely e.g., physiological and pathological brain conditions.

In such a 3D cell culture system it is now possible to compare and evaluate the effects of a substance of interest on the different neuronal cells or brain cells. It was previously shown that such brain cells without the exposure to any drug do show a response to the viscoelasticity of the hydrogel (Klostermeier et al., in press], which can be measured e.g. as gene activation and overexpression, with an assumed protein accumulation.

The response to differences in the viscoelasticity of the hydrogel was shown to resemble the situation in the human brain, where in e.g. injured brain the stiffness and elasticity of the extracellular matrix and/or tissue are amended by the influence of cytokines, infiltrating blood or other inflammatory factors. This influence leads to a more "stiff" tissue condition, which is imitated in the stiff test gels of the present invention.

Similarly, it was shown by the inventor that within the 3D cell culture system by comparing the 3D development of brain cells in the corresponding hydrogels with different viscoelasticity the development and metabolism of said brain cells depends on the viscoelasticity.

In other words, in a healthy brain, which has a viscoelasticity with a stiffness of around 1 kPa and shear modulus around of 0.4-1.4 kPa, as it is also provided in the "soft" hydrogel of the presently described 3D cell culture system, the cultivated brain cells develop in a physiological manner. If tested for e.g. the expression of certain genes, and in particular for so-called AD risk genes, or alternatively, if tested for the accumulation of Aβ or pTau, it was shown that in soft brain tissue and soft hydrogels no risk genes overexpression or accumulation was found.

However, in the hydrogel with the pathological viscoelasticity, which is throughout the application defined as stiff, and wherein the findings imitate and resemble tissue conditions of an AD affected brain, and wherein the cultivated cells behave differently and resemble morphological the pathological findings in AD, the results are different. It was found that in such stiff hydrogels the tested brain cells show clear overexpression of AD risk genes and/ do accumulate Aβ or pTau.

On the bases of these findings, it is now possible to test substances of interest on their effects on cultivated cells e.g. brain cells under different viscoelasticity conditions. For the AD research this allows for the first time to test substances of interest on their effect on brain cells under the pathophysiological condition of an AD brain and compare them to physiological conditions of the human brain.

It is particularly, the comparison between the at least first and at least second hydrogel of the inventive test system, wherein each hydrogel has a distinct and different viscoelasticity allowing deep insights into the development, the physiology and the pathology of different cells and tissues, and in case that such cells are exposed to a substance of interest it also allows to observe and evaluate the specific influence of said substance on the cells of interest.

Accordingly, the herein described 3D cell culture system is suitable to compare, illustrate and/or measure the effect of a substance of interest on the cultured cells in a pharmacological, toxicological, metabolic and/or dose-dependent manner.

Such testing in the 3D cell culture system as described herein can be made for individual cell types or collective cell agglomerates as found in tissue probes or primary tissues extracted in a biopsy.

Particularly, interesting for the present application is the selection of brain cells including neurons, glia cells, e.g. astrocytes, microglia cells and combinations thereof. Additionally, also suitable is the 3D cell culture system for the testing of cell lines derived from brain cells or stem cells differentiated into brain cells.

According to another embodiment of the 3D cell culture system and in one highly specific embodiment the cells to be cultivated and tested are selected neurons, glia cells, or combinations thereof. Additionally, such cells to be cultivated and tested can be selected from cell lines derived from brain cells, tissue samples and even biopsies derived from patient brains. Last not least, such cells to be cultivated and tested can be selected from stem cells, which under suitable and conditions known to the skilled person can differentiated into brain cells.

All such cells can be tested in the 3D cell culture system on the effect of **a substance of interest** on such brain cells by employing **a method for testing substances and/or compositions** which comprises the steps of firstly preparing of the 3D cell culture systems as described above with at least a first and a second hydrogel having selected but distinct viscoelasticity.

In a second step the hydrogels are populated with cells to be tested e.g. from the group of brain cells comprising, neurons, glia cells, microglia cells and combinations thereof. Alternatively, also cells selected from cell lines derived from brain cells, tissue samples or biopsies derived from patient brains or stem cells differentiated into brain cells have been selected for this method.

For this, the selected cells will be counted and then mixed into the pre-pared - still liquid - hydrogel solution by gently pipetting the cell-hydrogel mixture up and down until a homogeneous mixture of cells-hydrogel solution is created. By slowly increasing the temperature to conditions suitable for the cells to be tested, namely 32°C to 37°C, the hydrogel solution of the present invention starts polymerize and starts to build up the viscoelasticity predefined by the selection of the alginate preparation.

The cells can then be directly tested after mixing them into the hydrogel, or optionally can be cultivated for a defined period to establish e.g. 3D structures, which might be of interest for the testing.

The culturing of the brain cells or any other cell types in the at least first and the at least second hydrogel is performed under suitable conditions depending on the cell types. For comparing the results between the hydrogels with different viscoelasticity the cell culture conditions, besides the gel's biomechanics, are to be identical.

Consequently, after the culture conditions have been established in the next step of the method the cultivated cells in the at least first and the at least second hydrogel get exposed to at least one compound of interest. The exposure can be a one-time exposure or a repetitive exposure. Furthermore, the exposure can be performed by mixing the substance of interest into the hydrogel e.g. while preparing the gels, dripplingthe diluted substance to the hydrogel, by soaking the hydrogel or by flooding the hydrogel after the establishment of the test cells in the hydrogel.

Either right from the beginning of the exposure or after a defined time the cells are analyzed for differences regarding physiological and/or pathological changes. Such analysis cover for example - but is not limited to - tests for metabolic regulation, tests for morphology development, analysis of gene expression or RNA expression, as well as protein expression and/or protein accumulation. Other molecular biological relevant tests or analysis for identifying different reactions are of course suitable as well.

The data collected from such analysis, or the various tests are, optionally, extracted and prepared for external evaluation or AI-driven evaluation. Alternatively, the collected data are analyzed by comparing the data of a first hydrogel with the data of a second hydrogel, which is typically a negative (untreated) control, or alternatively a hydrogel with a different viscoelasticity.

Such comparison then allows the identification of substances that cause physiological and/or pathophysiological differences in the cells exposed to said substance. Such comparison further allows to identify substances which are capable of reversing on a cellular level a pathological condition into a normal or physiological condition.

With this new method and 3D cell culture system for the first time it is possible to not only observe the development of the pathophysiology of e.g. AD but - even more interesting - to identify substances and/or compositions, which interfere, also reduce and delay or even may revert the pathophysiology of AD. Such observations are e.g. intensified by testings whereby the cells of interest are exposed in one or several hydrogels of controlled or standardized stiffness (e.g. still hydrogels) to the substance of interest in comparison to a negative (untreated by the substance of interest) control or a physiological control being a hydrogel with physiological viscoelasticity and treated with the substance of interest.

Advantageously, by testing further substances more knowledge about the so far still not well understood pathomechanism of neurological disorders including the pathology of e.g. AD may be collected.

The main characteristic of the method and the 3D cell culture system herein described is that the 3D cell culture system comprises two or more hydrogels, which are made in a highly reproducible manner from primarily - but not limited to - size-defined ADA and collagen, whereby each hydrogel can be reproducible prepared in a distinct stiffness and elasticity and thus, viscoelasticity. While the viscoelasticity can be matched with a tissue specific viscoelasticity, and in this context also with the physiological and pathophysiological viscoelasticity specific for a defined tissue.

Alternatively, the individual hydrogels can also have gradually increasing or decreasing viscoelasticity, in other words having matching viscoelasticity.

Another characteristic the 3D cell culture system herein described is that the two or more hydrogels are free from any cytokines or free from any other cell stimulating or modulating substances, such as cytokines, growth factors or other inhibiting and/or activating factors. Accordingly, the method herein described and the cells growing in the 3D cell culture system are not affected by any such stimulating, inhibiting and/or activating factors.

The method herein described is particularly suitable for testing any compound of interest for identifying effects of such compounds regarding its pharmaceutical activity or its metabolical effects on the cells or tissues tested.

This is particularly helpful for testing already established pharmaceutical drugs and their potential of repurposing them for a treatment of a new therapeutical indication. Particularly interesting in this regard is the testing of pharmaceutical drugs selected from small molecules, antibodies, receptor ligands, biologics and/or biosimilars.

Further interesting is the testing of pharmaceutical drugs in comparison to the same drug in combination of a pharmaceutical composition comprising adjuvants, pharmaceutical acceptable carrier, diluents and/or excipients. Such comparison allows to characterize and distinguish the pharmaceutical effect form possible side effects caused by additional compounds in a pharmaceutical composition.

Even more interesting is the testing of a compound of interest on a target tissue when administered in combination with additional and further drugs a patient is taking or might have to take for other unrelated treatments. With such experimental setup, it is for the first time possible to effectively evaluate side effects of combination therapy on the healthy brain but also on already pathologically modified brain tissue. It is thus, further possible to identify also unwanted effects or side effects of drug combination while being used in parallel or in combination therapies, which would otherwise not be predictable or only show due to their side effects.

It goes without saying that the method and the 3D test system herein described has a tremendous potential for identifying not only side effects of pharmacological drugs or different combinations of pharmacological drugs, but interestingly, for the first time it became feasible to also analyze and test for side effects on the brain tissue and the brain cells of various drugs or combination thereof with or without normal nutrients (e.g. ethanol).

The method and the 3D cell culture test system herein described has thus the potential not only to replace many *in vitro* testings but also to reduce or replace preclinical animal testings.

Particularly in the context of brain research, where suitable animal models are rare, a method as presented herein capable of simulating brain tissue and simulating the physiological but also the pathological condition of the human brain is allowing a much quicker and more reliable testing of suitable substances or drugs for the treatment of neurological disorders.

With this system, which simulates *in vivo* conditions for the brain, it will also be possible to test for suitable amounts of a substance of interest regarding its therapeutically effective doses or even suitable or necessary doses regimes.

The results of such doses regime testing also allow to identify a dose, which is potentially lower than the effective treatment dose, but still shows a curing or stabilizing effect and thus can leed to the development of the long-term, low-level treatments for neurological disorders and neurodegenerative disorders.

Such long-term and/or low-level treatment is advantageously administrable as a prophylactic treatment avoiding the development of neurological disorders and diseases or reducing the risk of developing such neurological disorders and diseases, as it might exist e.g. for patients with a genetic predisposition e.g. for AD.

Additionally, it will become feasible to also prophylactically treat neurological disorders which dependent on or are caused by changes of the extracellular viscoelasticity of the CNS. One example for such a risk that will become prophylactically treatable are brain injuries such as concussion, trauma or encephalitis.

### Description of the Figures

In the context of the application and in the Figures the abbreviation T-gel stands for "test hydrogel" according to the invention.

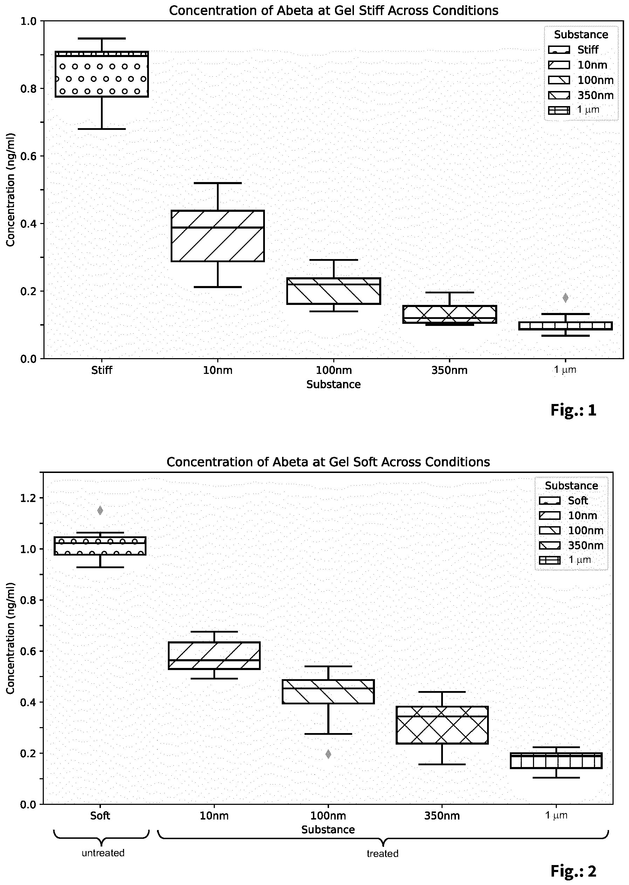

**Figure 1** shows the Abeta (hereinafter also identified as Ab or Aβ) protein concentration produced by human neuronal stem cells in a 3D in vitro microenvironment. The used test hydrogel (T-gel) "Stiff", emulating the AD microenvironment, shows Ab concentration with and without treatment by the test substance according to the invention as described in Example 1 as well as a treatment with different concentrations thereof. Drug concentrations in nanomolar (nm) and protein concentrations in nanogram/milliliter (ng/ml).
**Figure 2** shows the Ab protein concentration produced by human neuronal stem cells in a 3D in vitro microenvironment. The used hydrogel T-gel "Soft", emulating the healthy microenvironment, shows Ab protein concentration with and without treatment of the test substance according to the invention as described in Example 1 as well as a treatment with different concentrations thereof.

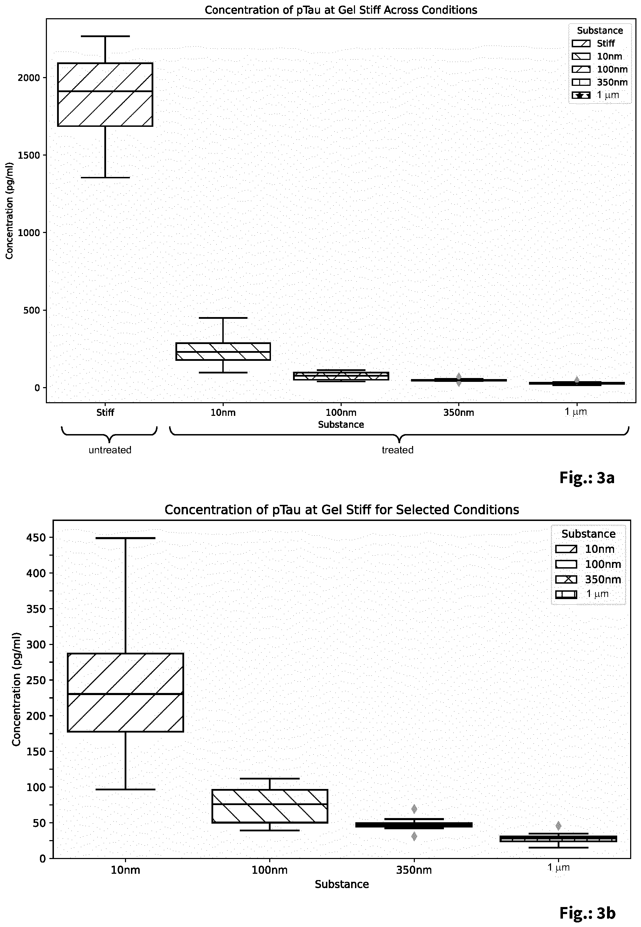

**Figure 3a** **und b** shows the pTau protein concentration produced by human neuronal stem cells in a 3D in vitro microenvironment. The used hydrogel T-gel "Stiff", emulating the AD microenvironment, shows pTau protein concentration with and without treatment of the test substance according to the invention as described in Example 1 as well as a treatment with different concentrations thereof.

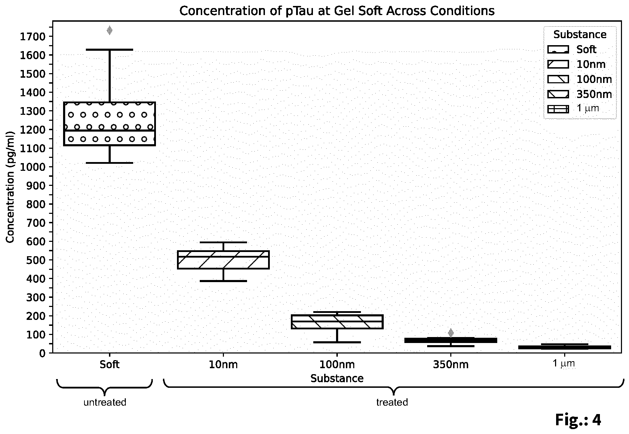

**Figure 4** shows the pTau protein concentration produced by human neuronal stem cells in a 3D in vitro microenvironment. The used hydrogel T-gel "Soft" shows pTau protein concentration with and without treatment of the test substance according to the invention as described in Example 1 as well as a treatment with different concentrations thereof.

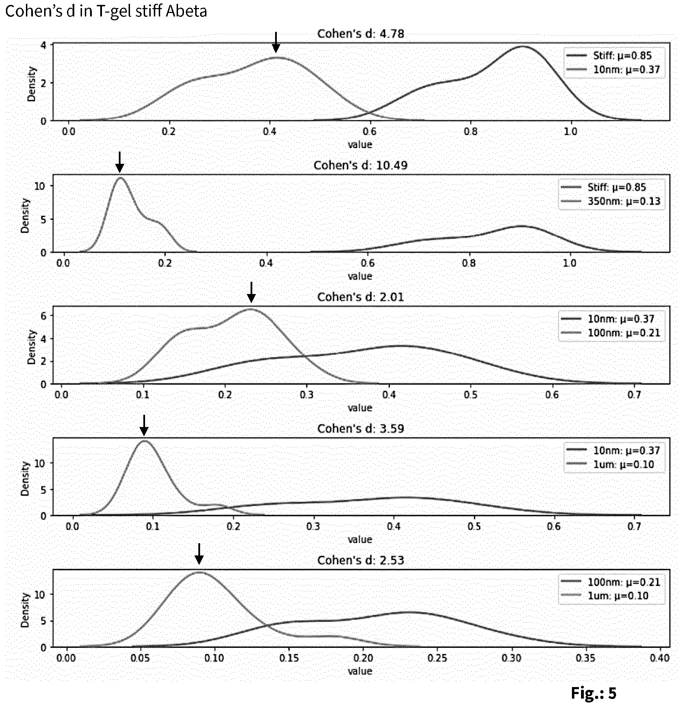

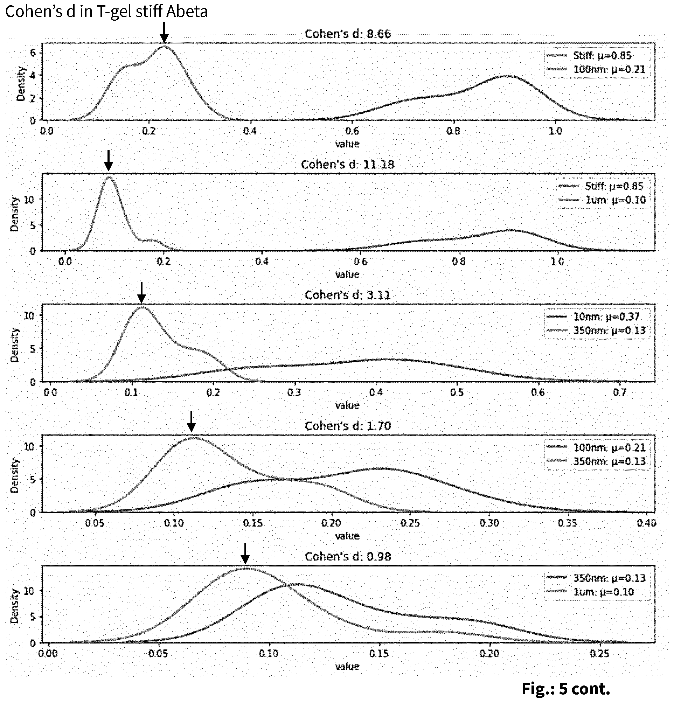

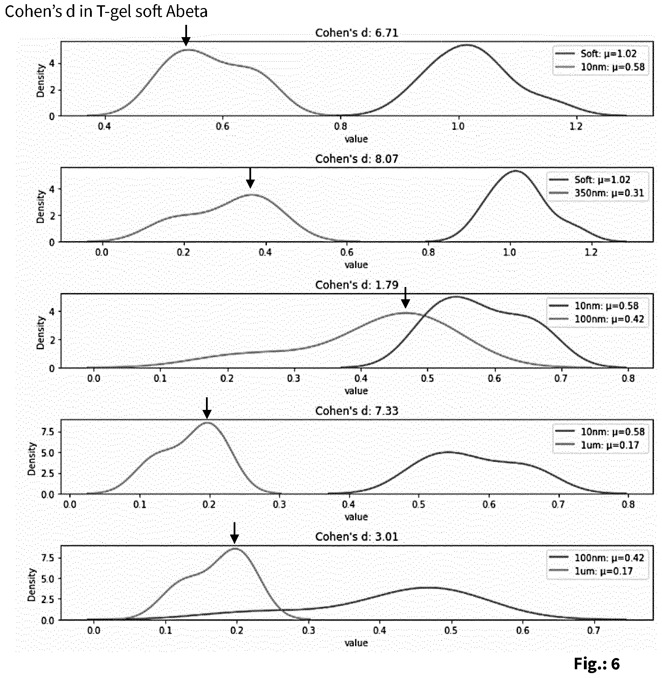

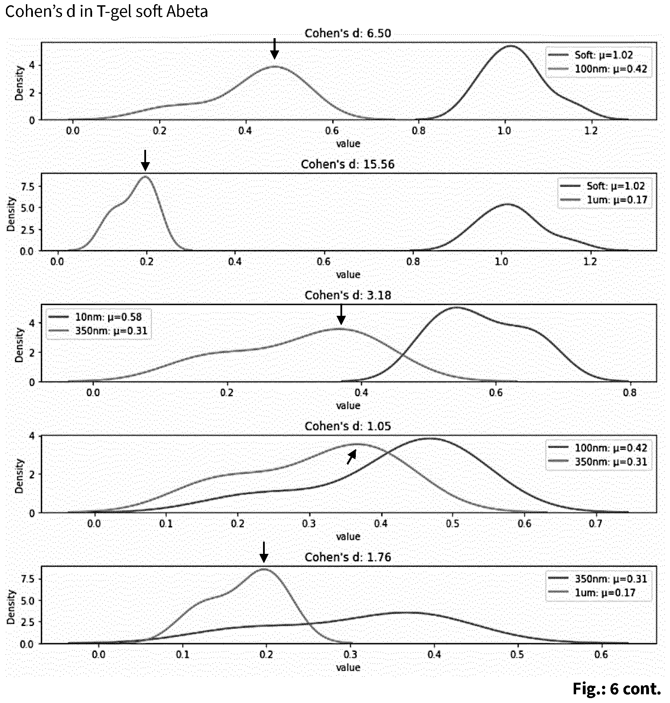

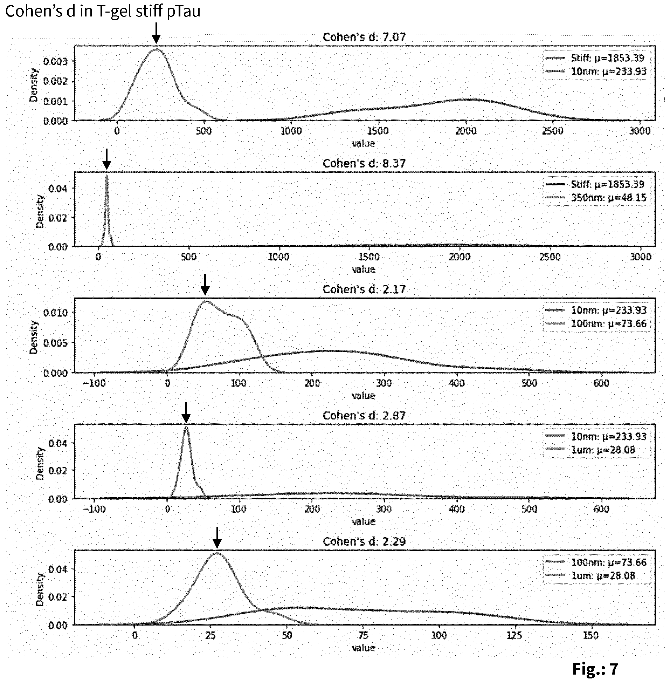

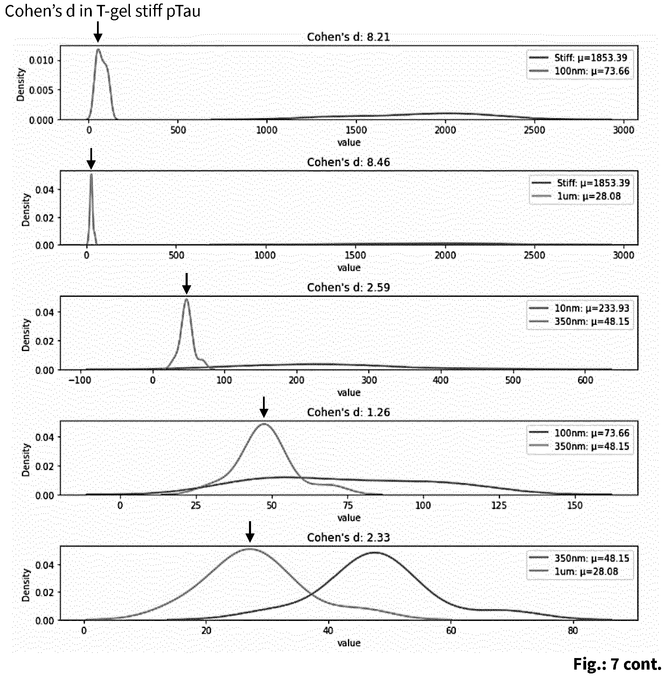

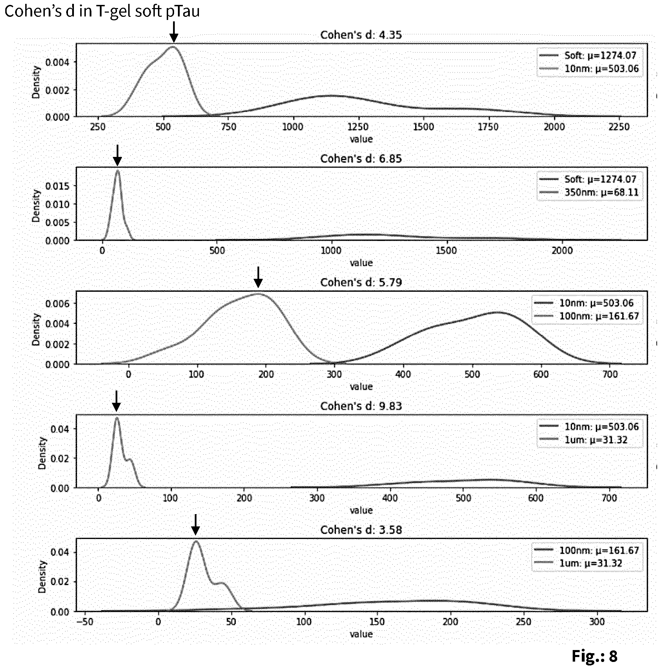

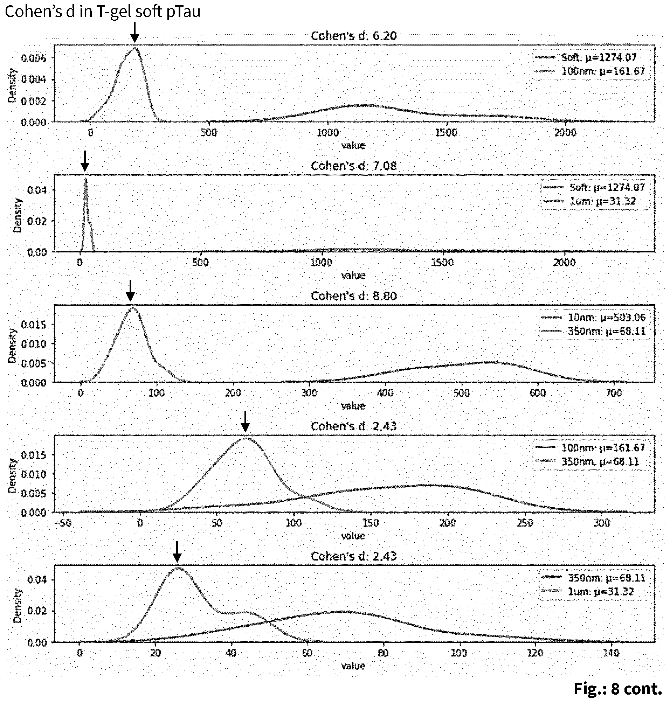

The figure legend for the Figures 5 to 8 are placed in the right upper corner of each single graph.

As can be seen in every single figure legend two curves are identified, which are comparing e.g. different substance concentrations used for the treatment in the test system. In the black and white illustration, in every graph each curve is identifiable via the position of it's maximum in the left or right part of the figure, respectively. The upper line-identifier of the figure legend refers to the curve having its maximum on the right part of the figure. The lower line of the figure legend refers to the curve having its maximum on the left part of the figure. Forfurther identification the maximum of the left curve is identifiable further by an additional arrow.

**Figure 5** shows the Cohen's d analysis corresponding with the results of Figure 1.

For Interpreting Cohen's d: The value of Cohen's d indicates the size of the effect, with higher absolute values indicating a larger effect. General benchmarks for interpretation, often used in scientific research, include: **Small** Effect Size: d=0.2; **Medium** Effect Size: d=0.5; **Large** Effect Size: d=0.8; These thresholds are guide lines and are here in the context of the research considered when interpreting the results.

**Figure 6** shows the Cohen's analysis corresponding with the results of Figure 2.

**Figure 7** shows the Cohen's analysis corresponding with the results of Figure 3.

**Figure 8** shows the Cohen's analysis corresponding with the results of Figure 4.

### Examples

### Example 1: Substance of interest: GDC-0077

Tested was a compound of formula **III,** which was diluted to the various concentrations. The different concentrations where mixed to the stiff and/or soft test gel preparations for testing.

### Example 2: Cells and cell lines for testing in the 3D cell culture system

### Human neuronal cell culture with human progenitor Alzheimer's disease cells

Expansion and differentiation media for ReNcell VM were prepared following previously established procedures (Nature protocols: A3D human neural cell culture system for modeling Alzheimer's disease DOI https://doi.org/10.1038/nprot.2015.065 ). ReN FAD (AD cells) (#SCC008FAD2, Alzheimer's In A Dish^{™} ReN-mGAP10, Clone D4 NSC Line, Merckmillipore, Germany) were subjected to expansion using ReNcell expansion media (#SCM005, Merckmillipore, Germany), including growth factors 20 ng/ml EGF (#GF144, Sigma, Germany), 20 ng/ml bFGF (#GF003, Sigma, Germany). The removal of growth factors from the ReNcell NCS Maintenance Medium leads to the spontaneous differentiation of ReNcell-immortalized neural progenitor cells. These cells were cultured on T25 or T75 cell culture flasks pre-coated with Matrigel (Merckmillipore, Germany). The cultures were maintained in a controlled environment with 5% CO₂ at 37 °C.

### Example 3: Establishing of test hydrogels (T-gel)

The test gel (T-gel) is a hydrogel composed of a polymer (ADA), collagen, and laminin as previously described in WO 2020/245302 A1.

The polymer basis of T-gel is ADA (alginate di-aldehyde) (PMID:32261366).

To achieve different biomechanical properties, the inventors modified the gel synthesis by using two different alginates, in this example PH124 and PH176 (VIVAPHARM JRS Pharma, Ulm, Germany). The ADA synthesis was performed as previously described Bapi Sarker et al., (J Material Chemistry, Issue 11, 2014) and takes approximately one week. Once synthesized the resulting polymer has a cotton-like structure and can be stored at room temperature in dry conditions.

For preparing the hydrogels, the selected ADA (here: PH124 or PH176) is dissolved in serum (4xOpti-MEM, ThermoFisher, Erlangen, Germany) followed by adjustment to pH 8.2 using sodium hydrogen carbonate and filter sterilization using a 0.22 µm filter (both from Roth, Karlsruhe, Germany).

The addition of 7.2 µl ADA solution, 15.8 µl collagen type I (Corning, Germany), 6 µl laminin (1.2 mg,

Corning, Germany), 0.4 µl NaHCO₃ (Roth, Germany), 0.3 µl Pen/Strep (Sigma, Germany), 4.6 µl H₂O to ADA at room temperature results in a liquid hydrogel stock solution, which solidifies with a temperature increase to 37 degree Celsius (T-GEL stock). To keep the T-gel stock solution liquid, the compounds are mixed on ice. The T-gel stock is used in a 1:10 solution with a standard culture medium to prepare the test gels.

### Example 4: Preparation of the 3D cell culture test system

For preparing the "soft" T-gel the stock solution made with ADA PH 123 is diluted 1:10 with cell culture medium and the prewashed test cell as herein described e.g. in Example 2 are mixed into the hydrogel preparation. Correspondingly, is the preparation of the "stiff" T-gel, where the only difference is the selection of ADA being PH176.

Additionally, the test substance in different concentrations where mixed to the stiff and soft test gels preparation.

Then the gel-cell-substance-mixture is then mixed with the suitable test cells and poured into suitable cell culture dishes for incubation in an incubator at 37°C and 5% CO2.

### Example 5: Test method for substance of interest

In a first step one cell culture system is prepared according to Example 4. This cell culture system comprises two hydrogels for the cultivation of suitable cells or cell lines.

For the present testings neurons, capable of expressing Abeta (Aβ) and/or pTau are used as described in Example 2. These cells are mixed with the corresponding hydrogel preparation and cultured in the two hydrogels under identical cell culture conditions.

In a next step the cultivated cells were exposed in the two hydrogels to the substance of interest, herein after substance of Formula III. For exposure the substance of interest is prepared in a suitable diluent with defined concentration, namely 0.0 nm (= untreated), 10nm (nanomolar), 100nm, 350nm, 1µm (micromolar). These preparations are then added onto the gel after the cells are already incubated in the gel. Alternatively, these preparations are mixed into the gel preparation together with the test cells while preparing the T-gels.

After exposure the test cells are analyzed regarding the physiological and pathological changes in response to the substance of interest. Such analysis covers e.g. test for metabolic regulation and morphology, effects due to the exposure to the substance of interest on gene expression, RNA expression, protein synthesis and protein accumulation.

Additionally, the amount of Ab protein or pTau produced from the test cells in the different test gels was analyzed and measured in ng/ml or pg/ml. The protein concentration of the untreated cells in comparison to cells treated with the test substance - here Formula III - is illustrated in Figure 1 and 2.

The comparison between the effects of the substance of interest on the cells in stiff or soft hydrogels as well as in hydrogels exposed or not-exposed to the substance of interest allows to identify substances which are suitable to effectively treat e.g. neurological disorders such as AD.

Consequently, the data presented, allow the comparison between the microenvironment of the healthy brain versus an AD brain, and additionally prove that the test substance of formula III has particularly in AD brain microenvironment positive effects on (i.e. showing a reduction of) the Aβ and pTau protein accumulation in neurons. Thus, the data included into this application show a substance suitable and promisingto reduce the accumulation of Aβ and pTau in neurons and thus, a first drug suitable and promisingto treatAD in humans.

### Example 6: Preparation of Aβ gels as control)

Briefly, lyophilized Aβ1-42 (Aggresure^{™} (AnaSpec) Fremont, USA) was dissolved in 1 ml NT-Gel/HEPES/pH 7.2 solution and plated in the gel preparation made from the 1:10 T-gel stock. The Aβ gels were incubated at 37 °C for 24 h.

### Example 7: Aβ Detection

### 7.1. Aβ analysis - Staining for analyzing the amount in the different test gels

For Aβ staining, thioflavin 5 staining 1% ThS solution was added and incubated for 15 min. Followed by a three times wash using PBS. Fluorescence signal (Ex 360 nm; Em 380 nm) was measured using a Axio Observer Z1 (Carl Zeiss).

### 7.2. Aβ analysis - Immunocytochemistry

Cells were treated in the test gels and thereafter isolated from the test gels and washed with PBS. Then the cells were fixed on plates or glass coverslips with 4% paraformaldehyde (Merck Millipore, #1040051000) for 15min at RT. They were washed twice with PBS.

The fixed cells were stored in PBS at RT or continued with ICC procedure. First, the cells were blocked and permeabilized for 1 hour at 37°C with 3% donkey serum (Pan Biotech, #P30-0101), 0.1% Triton X-100 (Sigma, #T8787) in PBS. Primary antibodies were diluted in blocking solution and the cells were stained for 2h at 37°C. The cells were washed twice with PBS and fluorescently labeled with secondary antibodies (Thermo Fisher Scientific) diluted in blocking solutions for 1 hour at 37°C.

The cell nuclei were stained for 2min at RT with 1µg/ml DAPI (Sigma, #32670). Afterwards, the slides were stored with PBS at RT until microscopy analysis. Images were taken with the Axio Observer Z1 fluorescence microscope (Carl Zeiss) and analyzed with the software ImageJ (version 1.52).

### 7.3. Aβ analysis - Gene expression & quantitative PCR

Primers according to Table 1 for qPCR were purchased from Integrated DNA Technologies (Leuven, Belgium) and are listed afterwards in Table 1 with sequences. In this context the sequence-ID's refer to a corresponding sequence listing for the present application.

**TABLE 1**

| **primer** | **sequence (5->3)** | **Sequence-ID** |
|---|---|---|
| Piezo1 forward primer | ATCGCCATCATCTGGTTCCC | **1** |
| Piezo1 reverse primer | TGGTGAACAGCGGCTCATAG | **2** |
| ADAM10 forward primer | CATGGTGAAACGCATAAGAATCA | **3** |
| ADAM10 reverse primer | CAACCCAAGCCAGACCAAGT | **4** |
| TREM2 forward primer | CACAGCATCTCCAGGAGCC | **5** |
| TREM2 reverse primer | GTGTCCCTGGCTTCTGTCC | **6** |
| GAPDH forward primer | AGGTCGGAGTCAACGGATTT | **7** |
| GAPDH reverse primer pair | TGGAATTTGCCATGGGTGGA | **8** |
| ADAM17 forward primer | GGCCGAATATAACATAGAGCCAC | **9** |
| ADAM17 reverse primer | TGAACAAGCTCTTCAGGTGGT | **10** |
| RHOH forward primer | CTTCGGCATTCTGCAACAGT | **11** |
| RHOH reverse primer pair | GCCTAGTCTTCAACTGGTGTG | **12** |
| BLNK forward primer pair | CCTCCTTGAGGATGAGGCTGA | **13** |
| BLNK reverse primer | CGACCTGAAGCTGTTCCTGG | **14** |
| SIGLEC11 forward primer | CACCCATTCAAATGGAGCACG | **15** |
| SIGLEC11 reverse primer | GCAGCTGTGGAGGGTAGTG | **16** |
| TRPV4 forward primer | CCCGGAGGCTGAGCA | **17** |
| TRPV4 reverse primer | TCAAACAGATTGGCCAGGGA | **18** |
| B2M forward primer | GAGTATGCCTGCCGTGTGAA | **19** |
| B2M reverse primer | CGGCATCTTCAAACCTCCAT | **20** |
| HPRT-1 forward primer | CCTGGCGTCGTGATTAGTGA | **21** |
| HPRT-1 reverse primer | CGAGCAAGACGTTCAGTCCT | **22** |

For RNA extraction, cells were plated as describes earlier in T-gel soft and T-gels stiff gels with or without Abeta. RNA was directly isolated according to manufacturer's instructions using the RNeasy Plus Universal Mini Kit (cat. #73404; QIAGEN, Hilden, Germany) and its concentration was determined with a NanoDropTM 2000 spectrophotometer (Thermo Fisher). cDNA was generated directly after RNA isolation according to manufacturer's instructions with High-Capacity cDNA Reverse Transcription Kit (cat. #4368814, Applied Biosystems) and then stored at -20 °C. RNA levels were determined using the ABsolute QPCR Mix (SYBR Green, no ROX, cat. #AB1158B, Thermo Scientific).

Quantitative PCRs were started using the RNA isolated as described above and the different primer pairs as mentioned above to analyze the amount of RNA expressed from the Alzheimer risk genes in response to the treatment with the substance of interest.

### 7.4. Aβ analysis - ELISA (Aβ and p-Tau)

For Aβ and p-Tau quantification 100 000 AD cells were plated in a 1:10 dilution of gel:medium and incubated.

ELISA analysis was prepared as described in the protocols (Aβ, Invitrogen, KHB3491; p-Tau,

Invitrogen, KHO0631). In brief, gels were washed with PBS (Brand, country) followed by centrifuging at 13 000 rounds per minute for 2 minutes. After repeating the last step, the gels were dissolved using 150 µl extraction buffer (Invitrogen, Germany, #FNN0011) including protease phosphatase inhibitor (Thermo Science, Germany, #A32961) vortexed, and incubated on ice for 30 minutes while vortexing every 10 minutes. Finally, the supernatant was used for protein quantification in the ELISA.

### Example 8: Neuron and microglia co-culture

For the co-culture experiments cell densities, with 100 000 cells per well for AD cell's (#SCC008FAD2, Alzheimer's In A Dish^{™} ReN-mGAP10, Clone D4 NSC Line, Merckmillipore, Germany) and ReN G2B2 WT (#SCC008, ReNcell VM humane neural Progenitor cell line, Merckmillipore, Germany) 33 000 cells per well for HMC3 were used.

T-Gels soft and stiff were used in a 1:10 dilution as described above. AD cells and WT neurons were plated in 1:10 T-Gels and incubated for 7 days at 37 °C, 5% CO2.

On day 8, 33 000 HMC3 microglia pre-labeled using Cell tracker violet BMQC dye (#C10094, Thermo Fisher scientific, Germany) cells were seeded on top of the T-Gel/neuronal cells. Microglia-neuron interactions were captured using Axio Observer Z1 (Carl Zeiss) for further analysis.

For testing the substance of interest was soaked into the different test gels or added onto the different test gels in variable concentrations. In this setup not only the direct effect of the therapeutic dose is recognizable, but also the interaction between microglia and neurons in response to the substance of interest is illustrated. The situation is thus even closer to the normal healthy human brain or an AD brain. Analytic testing according to the above described methods.

References:

Barker et al. (2020) "Mini Review: Opposing Pathologies in Cancer and Alzheimer's Disease: Does the PI3K/Akt Pathway Provide Clues", (Frontiers in Endocrinology, June 2020, Vol.11).

Bapi Sarker et al., (2014), "Fabrication of alginate-gelatin crosslinked hydrogel microcapsules and evaluation of the microstructure and physico-chemical properties", (J Material Chemistry, Issue 11,)

## Claims

1. A substance or composition for the use in the treatment of neurological disorders and/or Alzheimer Disease (AD) wherein the substance is a PI3K-inhibitor.

2. The substance or composition for use in the treatment of neurological disorders according
to claim 1, comprising a compound of formula **I,** 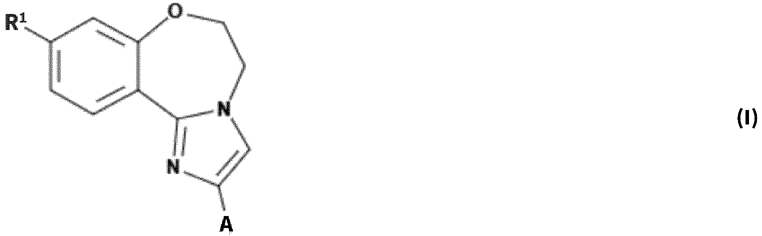
wherein the compound of formula I is a Benzoxazepine, a stereoisomer, constitutional isomer, geometric isomer, tautomer or pharmaceutically acceptable salt thereof; and wherein the compound of formula I has a first residue R¹ and a second residue A.

3. The substance or composition for the use according to claim 1 or 2 wherein
the first residue R¹ is 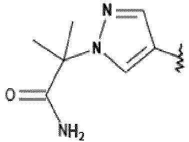 and wherein
the second residue A is 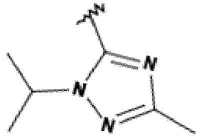 or wherein
the first residue R¹ is 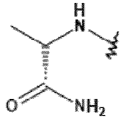 and wherein
the second residue A is

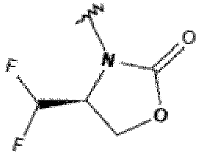

4. The substance or composition for the use according to any of the claims **1** to 3, wherein the neurological disorder is selected from the group comprising Alzheimer's Disease, Amyotrophic Lateral Sclerosis (ALS), Dementia, Vascular dementia, Frontotemporal dementia, Dementia with Lewy bodies, Parkinson's disease, Huntington's disease, progressive supranuclear palsy, corticobasal degeneration, multisystem atrophy, Down syndrome (DS), inflammatory bowel diseases, Crohn's disease, ulcerative colitis, meningitis and encephalitis, migraine, Creutzfeldt-Jakob disease and neuroborreliosis (Lyme disease), multiple sclerosis, neuromyelitis optica, Guillain-Barré syndrome, rheumatoid arthritis, systemic lupus erythematosus, epilepsies or seizure disorders, progressive myoclonic epilepsy, traumatic brain injuries, spinal cord injuries, neuromuscular disorders, myasthenia gravis, muscular dystrophies, spinal muscular atrophy, polyneuropathies and diabetic polyneuropathy.

5. The substance or composition for the use according to any of the claims 1 to 4, wherein the substance or composition is administered oral, intravenous, subcutaneous, intrathecal, sublingual, transbuccal, transdermal, nasal, pulmonal, rectal, as Antibody-Drug-complex and/or as liposomal nanoparticles.

6. The substance or composition for the use according to any of the previous claims 1 to 5,
wherein the substance or composition is administrable as part of a retard composition;
and/or
wherein the substance or composition comprises the compound of Formula **(I)** in a therapeutically effective amount.

7. The substance or composition for the use according to any of the previous claims 1 to 6, wherein the treatment is prophylactic or post-expositional prophylactic.
